(19)

Europäisches
Patentamt
European
Patent Office
Office européen
des brevets

(11) **EP 3 367 897 B1**

(12) **EUROPEAN PATENT SPECIFICATION**

(45) Date of publication and mention
of the grant of the patent:
**14.04.2021 Bulletin 2021/15**

(21) Application number: **16787481.7**

(22) Date of filing: **27.10.2016**

(51) Int Cl.:
**A61B 5/0452** *(2006.01)*　　　**A61B 5/0456** *(2006.01)*

(86) International application number:
**PCT/EP2016/075972**

(87) International publication number:
**WO 2017/072250 (04.05.2017 Gazette 2017/18)**

(54) **AN AUTOMATIC METHOD TO DELINEATE OR CATEGORIZE AN ELECTROCARDIOGRAM**

AUTOMATISCHES VERFAHREN ZUR DARSTELLUNG ODER KATEGORISIERUNG EINES
ELEKTROKARDIOGRAMMS

PROCÉDÉ AUTOMATIQUE POUR DÉLIMITER OU CATÉGORISER UN
ÉLECTROCARDIOGRAMME

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Validation States:
**MA**

(30) Priority: **27.10.2015 EP 15191769
27.10.2015 US 201514924239**

(43) Date of publication of application:
**05.09.2018 Bulletin 2018/36**

(73) Proprietor: **Cardiologs Technologies
75647 Paris Cedex 13 (FR)**

(72) Inventors:
 • **RAPIN, Jérémy
 75014 Paris (FR)**
 • **LI, Jia
 75006 Paris (FR)**
 • **MASSIAS, Mathurin
 75014 Paris (FR)**

(74) Representative: **Vossius & Partner
Patentanwälte Rechtsanwälte mbB
Siebertstrasse 4
81675 München (DE)**

(56) References cited:
 **CN-A- 104 970 789　　US-A1- 2013 237 776**

 • **Jin Linpeng: "Electrocardiogram classification
 method and system, abstract of CN104970789", ,
 14 October 2015 (2015-10-14), XP055264877,
 Retrieved from the Internet: URL:epodoc
 [retrieved on 2016-04-13]**
 • **KIRANYAZ S ET AL: "Convolutional Neural
 Networks for patient-specific ECG
 classification", 2015 37TH ANNUAL
 INTERNATIONAL CONFERENCE OF THE IEEE
 ENGINEERING IN MEDICINE AND BIOLOGY
 SOCIETY (EMBC). PROCEEDINGS IEEE
 PISCATAWAY, NJ, USA, August 2015 (2015-08),
 pages 2608-2611, XP002756380, ISBN:
 978-1-4244-9270-1**
 • **SERKAN KIRANYAZ ET AL: "Real-Time
 Patient-Specific ECG Classification by 1-D
 Convolutional Neural Networks", IEEE
 TRANSACTIONS ON BIOMEDICAL
 ENGINEERING., vol. 63, no. 3, 14 August 2015
 (2015-08-14), pages 664-675, XP055264879,
 PISCATAWAY, NJ, USA. ISSN: 0018-9294, DOI:
 10.1109/TBME.2015.2468589**
 • **MEGHRICHE ET AL.: "On The Analysis of a
 Compound Neural Network for Detecting Atrio
 Ventricular Heart Block (AVB) in an ECG Signal",
 INTERNATIONAL JOURNAL OF MEDICAL,
 HEALTH, BIOMEDICAL, BIOENGINEERING AND
 PHARMACEUTICAL ENGINEERING, vol. 2, no. 3,
 2008, pages 68-78, XP002765772,**

**Description**

**FIELD OF INVENTION**

The present invention relates to temporal signal analysis, preferably cardiac signal analysis, more preferably electrocardiogram analysis, using at least one neural network.

**BACKGROUND OF INVENTION**

Electrocardiogram (ECG) and endocardiogram are graphic representations of the electrical activity of the heart. Electrocardiogram is recorded from the body using a number of electrodes placed in specific predefined areas. It is considered as a fundamental tool of clinical practice. It is a simple, non-invasive exam that can be performed by any health professional. Placing the electrodes is not considered as a medical procedure, yet in some countries, the prescription of the ECG by a doctor is essential for it to be performed. It is known that the ECG constitutes the first step in cardiovascular diseases (CVD) diagnosis, and is used multiple times throughout the life of a CVD patient. CVD constitute the first global cause of death.

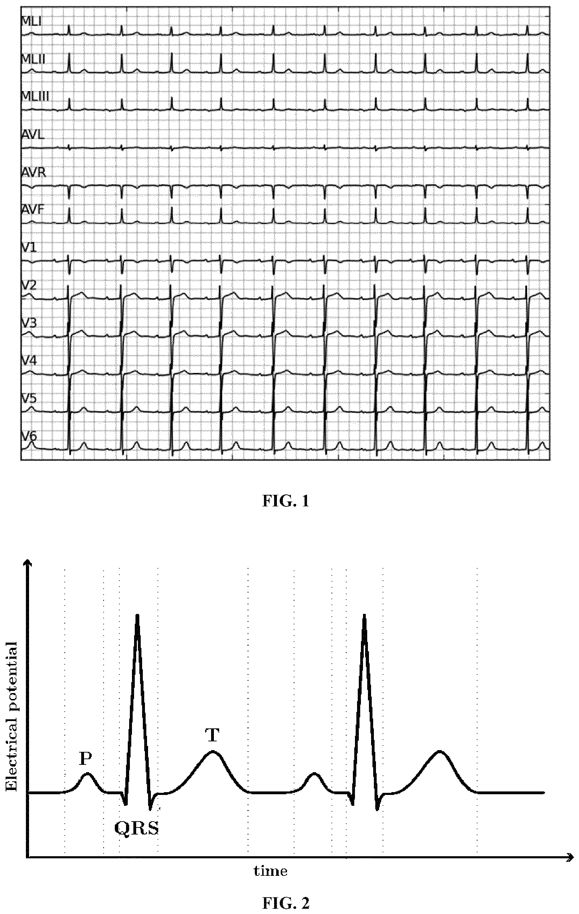

A cardiac signal is composed of one or multiple synchronized temporal signals, called lead signals. The ECG shown in figure 1 represents a standard 12-lead resting ECG, with its 12 standard deviations recording during 10 seconds. Some ECG, specifically known as Holters, may record only one lead for a period of time which can be of more than 7 days.

A cardiac signal displays repeating patterns usually comprising a P-wave, a QRS complex and a T-wave, respectively corresponding to the depolarization of the atria, depolarization of the ventricles and repolarization of the ventricles. These waves and complexes are shown in figure 2, which focuses on a couple of beats in one lead signal.

Cardiac signals allow for the detection of many abnormalities, which often in turn point to specific CVD. It is estimated that about 150 measurable abnormalities can be identified on an ECG recordings today. However, without specific expertise and/or regular training, only a small portion of these abnormalities can be easily spotted. Unfortunately, today, it is estimated that only one third of ECGs are performed in settings where cardiology expertise is readily available.

In order to make cardiac signal interpretation, especially ECG interpretation simpler and assist non-specialists, two alternatives exist today, but neither fully satisfy the needs of health professionals:

- Telecardiology centers, where an interpretation of an ECG sent by a non-specialist is delivered either by a cardiologist or by a specialized ECG technician. Their interpretations are of high quality but are slow and expensive to obtain.
- Prior art automated cardiac signal interpretation softwares, which are mainly developed by cardiac signal device manufacturers. They provide low quality interpretation (false alarms are very frequent) but deliver them in seconds.

Prior art automated cardiac signal interpretation softwares can provide two types of information about a cardiac signal:

- a local information called delineation, providing the temporal location of each wave and optionally qualifying each wave separately; and/or
- a global information providing a classification of the cardiac signal as normal/abnormal or labeling its abnormalities.

Concerning delineation, two main approaches are used for finding the waves of cardiac signals.

The first one is based on multiscale wavelet analysis. This approach looks for wavelet coefficients reaching predefined thresholds at well-chosen scales (Martinez et al, IEEE transactions on biomedical engineering, Vol. 51, No. 4, April 2004, 570-581, Almeida et al., IEEE transactions on biomedical engineering, Vol. 56, No. 8, August 2009, pp 1996-2005, Boichat et al., Proceedings of Wearable and Implantable Body Sensor Networks, 2009, pp 256-261, US 8,903,479, 2014-12-02, Zoicas et al.). The usual process is to look for QRS complexes, and then look for P waves on the signal before the complexes, and after them for T waves. This approach can only handle a single lead at a time, sometimes using projection to one artificial lead (US 2014/0148714 - 2014-05-29, Mamaghanian et al.). This computation is made very unstable by the use of thresholds. The approach is also limited as it can neither deal with multiple P waves nor with "hidden" P waves.

The second one is based on Hidden Markov Models (HMM). This machine learning approach considers that the current state of the signal (whether a sample is either part of a QRS complex, a P wave, a T wave or no wave) is a hidden variable that one wants to recover (Coast et al., IEEE transactions on biomedical engineering, Vol. 37, No. 9, September 1990, pp 826-836, Hughes et al., Proceedings of Neural Information Processing Systems, 2004, pp 611-618, US 8,332,017, 2012-12-11, Trassenko et al.). To this end, a representation of the signal must be designed using handcrafted "features", and a mathematical model must be fitted for each wave, based on these features. Based on a sufficient number of examples, the algorithms can learn to recognize each wave. This process can however be cumbersome since

2

the feature design is not straightforward, and the model, usually Gaussian, is not well adapted. Also, none of these works has considered the situation of hidden P waves.

In the state-of-the-art, characterization of the waves for the delineation is only performed on the QRS to detect for instance ventricular or paced beats, and done in a second step, once the waves have already been localized. Such methods usually use standard classification algorithms which learn the type of beat based on many training examples of handcrafted set of features and corresponding beat label (Chazal et al., IEEE Transactions on Biomedical Engineering, 2004, vol. 51, pp. 1196-1206). These methods are limited in that the features which have been handcrafted will always be suboptimal since they were not learnt and may have erased some crucial information.

In order to solve the above issues, the latest works (Kiranyaz et al, IEEE Transactions on Biomedical Engineering, 2016, Vol. 63, pp 664-675) have turned to novel architectures called neural networks which have been intensively studied and had great results in the field of imaging (Russakovsky et al., arXiv:1409.0575v3, 30 January 2015). Indeed, these methods bypass the need of handcrafted features and directly learn from raw or mildly preprocessed data. Still, these applications of neural networks to cardiac signal waves characterization are very limited since:

- they must first rely on an algorithm able to detect the waves;
- they were only developed for QRS characterization; and
- they lack context information in processing one beat at a time, surrounding beats often providing important information.

Concerning abnormalities and/or CVD detection, most algorithms use rules based on temporal and morphological indicators computed using the delineation: PR, RR and QT intervals, QRS width, level of the ST segment, slope of the T wave, etc... These rules such as the Minnesota Code (Prineas et al., Springer, ISBN 978-1-84882-777-6, 2009) were written by cardiologists. However, they do not reflect the way the cardiologists analyze the ECGs and are crude simplifications. Algorithms such as the Glasgow University Algorithm are based on such principles (Statement of Validation and Accuracy for the Glasgow 12-Lead ECG Analysis Program, Physio Control, 2009).

More advanced methods use learning algorithms, and are built using a diagnosis and an adequate representation for each cardiac signal they learn from. In, Shen et al., Biomedical Engineering and Informatics (BMEI), 2010, vol. 3, pp. 960-964 for instance, the author used support vector machines to detect bundle branch blocks. However, in these methods, once again, it is necessary to seek a representation of the raw data into a space that preserves the invariance and stability properties. Indeed, cardiac signals vary significantly from one patient to another. It is therefore extremely difficult for an algorithm to learn how to discriminate different diseases by simply comparing raw data. A representation which drastically limits this interpatient variability while preserving the invariance within the same disease class must be chosen. Also, once again these representations usually rely on a preliminary detection of the beats and hence in a reliable delineation.

Some scientific teams very recently also turned to neural network architectures, but limitations still arose when they attempted to apply them to ECGs.

One team (Jin and Dong, Science China Press, Vol. 45, No 3, 2015, pp 398-416; CN104970789) proposed binary classification on a full ECG, hence providing one and only one class for any analyzed ECG. This is for instance a classification normal Vs abnormal (see [0027] of CN104970789). Their architecture use convolutional layers which process the leads independently before mixing them into fully connected layers. The authors also mention multi-class analysis, aiming at recovering one class among several, but they do not consider the less commonly used multi-label classification, which is however crucial in ECG analysis since one ECG can have several abnormalities such as for instance a left bundle branch block with atrial fibrillations.

Thus, there is a need for methods able to analyze cardiac signal, especially ECG, that can:

- carry out the analysis without the need for beat-by-beat processing, or feature extraction;
- obtain the delineation of the signal, including identification of hidden P waves, qualification of each wave in a single step, and optionally present this information in an comprehensible way;
- provide a multi-label classification directly from at least one time window of a cardiac signal, generally exhibiting multiple labels, contrary the prior art, which provides a single exclusive label;
- process data with varying number of leads with a same neural network;
- be fast, stable and reliable.

## SUMMARY

To address the above issues in cardiac signal analyses, the Applicant developed two techniques based on convolutional neural networks:

- A convolutional neural network which first gives a dense prediction of the probability of presence of each wave on each time stamp of the cardiac signal, then post-processes the signal to produce its delineation. This novel approach of the delineation, using convolutional networks, allows the processing of cardiac signals of any duration, analyzing and qualifying all types of waves in the same way in a single step, without being constrained by their positions.

- A convolutional neural network which directly predicts multiple labels on the cardiac signal. It results in a fixed format multi-label output which can represent abnormalities such as for instance "Atrial fibrillations" or descriptors such as for instance "Normal sinus rhythm" or "Noisy ECG".

Thus, the present invention is defined in claim 1 and relates to a computer implemented method for delineation and multi-label classification of a cardiac signal having a plurality of time points, said method comprising: - receiving the cardiac signal, the cardiac signal generated by a plurality of electrocardiogram (ECG) leads sensing over a plurality of heartbeats; - applying a first convolutional neural network to said cardiac signal, wherein the first convolutional neural network: • reads each time point of the plurality of time points assigned to the cardiac signal; • analyzes spatio-temporally each time point of the cardiac signal; • assigns to each time point of the cardiac signal a plurality of first scores indicative of the presence of a plurality of types of waves comprising at least the following types of waves: P-waves, QRS complexes, T-waves; • computes an onset and an offset of each type of wave of the plurality of types of waves in the cardiac signal based on the plurality of first scores assigned to each time point; • computes a plurality of global measurements based on the onsets and the offsets; and - applying a second convolutional neural network to said cardiac signal, wherein the second convolutional neural network: • reads each time point of the cardiac signal and the plurality of global measurements; • analyzes each time point of the cardiac signal and analyzes the plurality of global measurements; • computes a plurality of second scores for a time window aggregating at least two time points; and • allots to the time window a plurality of labels which each have a score higher than at least one predetermined threshold.

Further aspects of the present invention are defined in the dependent claims.

## DEFINITION

**"Abnormality"** refers to any physiological abnormality which can be identifiable on the cardiac signal. Today about 150 measurable abnormalities can be identified on cardiac signal recordings. For instance, within the present invention, the following abnormalities may be nonlimitatively identified: "Sinoatrial block, paralysis or arrest", "Atrial Fibrillation", "Atrial fibrillation or flutter", "Atrial Flutter", "Atrial tachycardia", "Junctional tachycardia", "Supraventricular tachycardia", "Sinus tachycardia", "Ventricular tachycardia", "Pacemaker", "Premature ventricular complex", "Premature atrial complex", "First degree atrio-ventricular block (AVB)", "2nd degree AVB Mobitz I", "2nd degree AVB Mobitz II", "3rd degree AVB", "Wolff-Parkinson-White syndrome", "Left bundle branch block", "Right bundle branch block", "Intraventricular conduction delay", "Left ventricular hypertrophy", "Right ventricular hypertrophy", "Acute myocardial infarction", "Old myocardial infarction", "Ischemia", "Hyperkalemia", "Hypokalemia", "Brugada", "Long QTc", etc...

**"Cardiac signal"** refers to the signal recording the electrical conduction in the heart. Said cardiac signal may be for instance an electrocardiogram (ECG) or an endocardiogram. Such signals may have one or more channels, called leads. It may be short term (10 seconds in standard ECGs) or long term (several days in Holters).

**"Classification"** refers to the task of categorizing objects into a list of groups. Such a task includes for instance recognizing the animal from a picture (the list of groups is then a list of animals), or recognizing whether an ECG is normal or abnormal.

**"Multi-label classification"** refers to identifying objects as being part of none, one or several groups of a given list of groups. Such a task includes for instance identifying none to several animals from a picture, or identifying none to several abnormalities on an ECG.

**"Delineation"** refers to the identification of the temporal localization of each of the waves of a cardiac signal. Delineation can also optionally provide more precise characterization of each of the waves.

**"Descriptor"** refers to a description of a cardiac signal which is not an abnormality, such as for instance "Normal ECG", "Normal sinus rhythm" or "Noisy cardiac signal", "Electrode inversion", etc...

**"Hidden P wave"** refers to a P wave which occurs during another wave or complex, such as for example during a T wave.

**"Label"** refers to a class used within the present invention for multi-label classification of a cardiac signal. Said label can be an abnormality or a descriptor. Labels are none exclusive. For instance, one can observe an Atrial fibrillation and Wolff-Parkinson-White together.

**"Delineation-based labels"** refers to labels which can be deduced (i.e. computed) from the delineation and its measurements. For instance, within the present invention, the following delineation-based labels may be nonlimitatively: "short PR interval" (PR interval < 120ms), "First degree AV block" (PR interval > 200ms), axis deviations, "Long QTc", "Short QTc", "Wide complex tachycardia", intraventricular conduction blocks, etc...

**"Local measurements"** refers to measurements directly derived from the delineation, such as for instance a given RR interval (duration between one QRS complex and the following).

**"Global measurements"** refers to measurements derived from the delineation and aggregated through time, such as for instance a mean or median values of PR interval (duration between the beginning of a conducted P wave and the following QRS complex), P duration, QRS duration, QRS axis, median QT interval, corrected QT inverval (Qtc), corrected JT interval, heart rate, ST elevation, Sokolov index, number of premature ventricular complex, number of premature atrial complexes, ratio of non-conducted P waves, ratio of paced waves etc...

**"Neural network"** refers to a mathematical structure taking an object as input and producing another object as output though a set of linear and non-linear operations called layers. Such structures have parameters which can be tuned through a learning phase so as to produce a particular output, and are for instance used for classification purposes. The input is then the object to categorize, and the output the probabilities to pertain in each of the categories.

**"Convolutional neural network"** refers to a neural network which is partly composed of convolutional layers, i.e. layers which apply a convolution on their input.

**"Fully convolutional neural network"** refers to a convolutional neural network in which all linear operations are convolutions.

**"Recurrent convolutional neural network"** refers to a particular convolutional neural network structure able to keep a memory on the previous objects it has been applied to.

**"Lead invariant structure"** refers to a structure proposed by the applicant to be able to use a same neural network for signals with any number of channels. Said structure is preferably used for neural networks processing Holters but not for networks processing standard 12 lead ECGs.

## DETAILED DESCRIPTION

The present invention relates to temporal signal analysis, preferably cardiac signal analysis, using at least one convolutional neural network.

According to one embodiment, the cardiac signal is recorded from any number of leads during from 1 second to several days.

According to one embodiment, the cardiac signal is recorded from 12 leads or more. According to an alternative embodiment, the cardiac signal is recorded from strictly less than 12 leads.

According to one embodiment, the cardiac signal is recorded from 12 leads or more under direct medical supervision (resting ECG, stress test, etc.).

According to an alternative embodiment, the cardiac signal is recorded from strictly less than 12 leads or not under direct medical supervision (ambulatory monitoring, etc.).

The framework used here is the one of supervised learning. The aim of supervised learning is to predict an output vector Y from an input vector X. In the Applicant embodiment, X is a cardiac signal (a multivariate signal) as a matrix of size **m** x **n**. As for Y, in the Applicant embodiment, it can be:

- the delineation, providing a score for each sample of X to be part of one of the different waves as a matrix of size **p** x **n**;
- the scores for each label as a vector of size **q**;
- the set composed of both the delineation and the vector of scores.

The problem of supervised learning can also be stated as follows: designing a function f such that for any input X, $f(X) \approx Y$. To this end, the function f is parametrized, and these parameters are "learned" (parameters are optimized with regards to an objective loss function, for example, by means of a gradient descent (Bishop, Pattern Recognition and Machine Learning, Springer, 2006, ISBN-10: 0-387-31073-8).

A neural network is a particular type of function f, aiming at mimicking the way biological neurons work. One of the most basic and earliest neural network is the perceptron (Rosenblatt, Psychological Review, Vol. 65, No. 6, 1958, pp 386-408). From the input X, it computes linear combinations (i.e. weighted sums) of the elements of X through a multiplication with a matrix W, adds an offset b, and then applies a non-linear function $\sigma$, such as for instance a sigmoid, on every element of the output:

$$f(X) = \sigma(WX + B)$$

The parameters which are learned in a perceptron are both W and B. In practice, more general neural networks are just compositions of perceptrons:

$$f(X) = \sigma_n(W_n \ldots \sigma_n(W_1 X + B_1) + B_n)$$

The output of a perceptron can be sent as input to another one. The input, the final output, and the intermediate states are called layers. The intermediate ones are more specifically called hidden layers, since only the input and the final output are observed. For instance, a neural network with one hidden layer can be written as:

$$f\,(X) = \sigma_2(W_2\,\sigma_1(W_1\,X + B_1) + B_2)$$

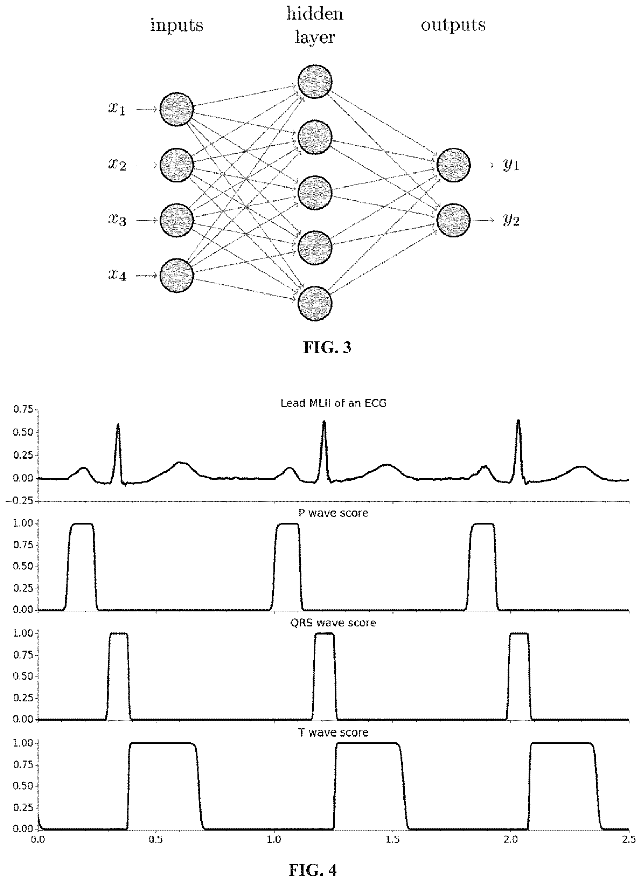

Such a network is shown in a graphic form as an example in Figure 3. The vector X enters the network as the input layer, each element of the hidden layer is then computed from linear combinations of all elements of X (hence all the links), and the element of the output layer are then computed from linear combinations of all elements of the hidden layer.

It has been shown that neural networks in their general form are able to approximate all kinds of functions (Cybenko, Math. Control Signals Systems, Vol. 2, 1989, pp 303-314). The term "deep learning" is used when a neural network is composed of many layers (though the threshold is not perfectly defined, it can be set to about ten). This field arose mostly in the last decade, thanks to recent advances in algorithms and in computation power.

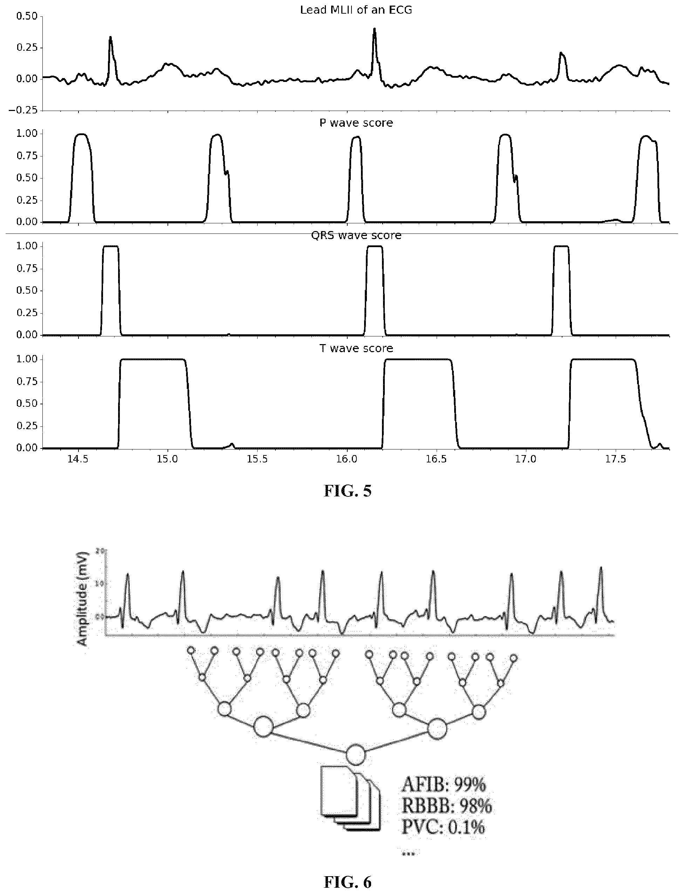

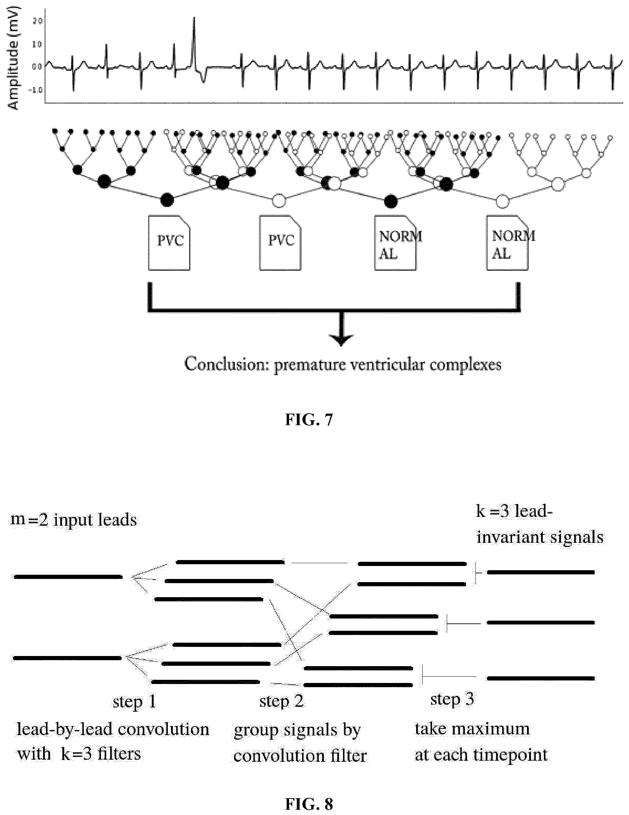

Convolutional neural networks are a particular type of neural networks, where one or more of the matrices $W_i$ which are learned do not encode a full linear combination of the input elements, but the same local linear combination at all the elements of a structured signal such as for example an image or, in this specific context, a cardiac signal, through a convolution (Fukushima, Biol. Cybernetics, Vol. 36, 1980, pp 193-202, LeCun et al., Neural Computation, Vol. 1, 1989, pp 541-551). An illustration of a convolutional neural network is shown in Figure 6. Most convolutional neural networks implement a few convolutional layers and then standard layers so as to provide a classification. A network which only contains convolutional networks is called a fully convolutional neural network. Finally, a recurrent convolutional neural network is a network composed of two sub-networks: a convolutional neural network which extracts features and is computed at all time points of the cardiac signal, and a neural network on top of it which accumulates through time the outputs of the convolutional neural network in order to provide a refined output. An illustration of a recurrent convolutional neural network is provided in Figure 7.

As mentioned above, a cardiac signal, especially an ECG is represented as a matrix of real numbers, of size **m** x **n**. The constant **m** is the number of leads, typically 12, though networks can be taught to process cardiac signal with any number of leads, as detailed herebelow. The number of samples **n** provides the duration of the cardiac signal **n** / **f**, with **f** being the sampling frequency of the cardiac signal. A network is trained for a given frequency, such as for example 250Hz or 500Hz or 1000Hz, though any frequency could be used. A same network can however process cardiac signal of any length **n,** if it is fully convolutional or a recurrent neural network.

In both the delineation and the multi-label classification embodiments, networks are expressed using open softwares such as for example Tensorflow, Theano, Caffe or Torch. These tools provide functions for computing the output(s) of the networks and for updating their parameters through gradient descent. The exact structure of the network is not extremely important. Preferred choices are fully convolutional networks in the situation of the delineation network (Long et al., Proceedings of Computer Vision and Pattern Recognition, 2015, pp 3431-3440), convolutional (Krizhevsk et al., Proceedings of Neural Information Processing Systems, 2012, pp 1097-1105) in the situation of the multi-label classification network, or recurrent neural networks (Donahue et al., arXiv:1411.4389v3, 17 Feb 2015 and Mnih et al., arXiv:1406.6247v1, 24 Jun 2014) for both the multi-label classification network and the delineation network. The 2D convolutional layers which were used on images are then easily converted into 1D convolutional layers in order to process cardiac signals.

In one embodiment, the network is amended to process data with varying number of leads in entry. In one embodiment, the neural network further comprises a sequence of layers at the beginning of the network so as to obtain a network which is independent of the number of input leads and can therefore process cardiac signals with any number of leads m. Such a structure is presented in figure 8 with m=2 input leads and k=3 output signals. The same structure can process any number of input leads m and will still provide k=3 signals in output, which can be fed to the rest of the network for which a fixed number of input signals is required. In this way, m need not be fixed anymore. According to one embodiment, in order to obtain a k-lead signal from an m-lead cardiac signal, the m leads are convoluted using a lead-by-lead convolution with k filters, the signal are then grouped by convolution filter in order to obtain k groups of m leads and a mathematical function is finally apply to each group to obtain k leads. According to one embodiment, any number of outputs k can be chosen. According to one embodiment, any number of inputs m can be used. According to one embodiment, the mathematical function is the maximum at each time point or may be any other function known to one skilled in the art. According to the Applicant, this feature was never disclosed before.

This invention also pertains to a method for manufacturing a neural network for delineation of a cardiac signal, by training it.

The training phase of the neural networks in the embodiment of delineation consists in the following steps:

- taking one cardiac signal from a dataset containing cardiac signals and their known delineation; the cardiac signal being expressed as a matrix of size **m** x **n** with m fixed and at a predefined frequency;
- expressing the delineation of this cardiac signal under the form of a matrix y of size **p** x **n** where **p** is the number of annotated types of wave; typically p=3, so as to identify P waves, QRS complexes, and T waves; annotations are expressed as lists of wave with their start and end points such as for example: (P, 1.2s, 1.3s), (QRS 1.4s 1.7s), (T, 1.7, 2.1), (P, 2.2, 2.3); in this example, the first row of y, corresponding to P waves, will be 1 for samples corresponding to times between 1.2 and 1.3s, and between 2.2 and 2.4s, and 0 otherwise; row 2 will correspond to QRS complexes and row 3 to T waves;
- computing the output of the network for this cardiac signal;
- modifying the parameters of the network so as to decrease a cost function comparing the known delineation and the output of the network; a cross-entropy error function is used so as to allow for multi-labeling (allowing for multiple waves at a given instant); this minimization can be done though a gradient step
- repeating steps 1 to 4 at least once for each cardiac signal of the dataset;
- recovering the neural network.

According to one embodiment, delineation further comprises wave characterization. According to said embodiment, p is the number of annotated types of wave plus the number of wave characterizations; for instance p=3+6=9 for identifying P waves, QRS complexes, and T waves, and characterizing premature waves, paced waves, ventricular QRS complexes, junctional QRS complexes, ectopic P waves and non-conducted P waves. According to said embodiment, annotations are expressed as lists of wave with their start and end points and characteristics such as for example: (P, 1.2s, 1.3s, [non-conducted]), (QRS 1.4s 1.7s, [premature, ventricular]), (T, 1.7, 2.1), (P, 2.2, 2.3); in this example, the first row of y, corresponding to P waves, will be 1 for samples corresponding to times between 1.2 and 1.3s, and between 2.2 and 2.4s, and 0 otherwise; row 2 will correspond to QRS complexes, row 3 to T waves, and row 4 corresponding to the premature characterization will be 1 during the premature QRS complex and 0 otherwise.

This invention also provides a method for manufacturing a neural network for the categorization of a cardiac signal, by training it.

In a multi-label classification, the manufacturing/training process includes the following steps:

- taking one cardiac signal from a dataset containing cardiac signals and their known labels; the cardiac signal must be expressed as a matrix of size **m** x **n** with m fixed and at a predefined frequency;
- expressing the labels as a vector of size q, with q the number of labels to identify; this vector could be [0; 1; 0; 0; 1; 0; 0; 0] for q=8; a 1 is set in the vector at the index corresponding to the labels which are present (i.e. having a score above at least one predefined threshold such has for instance 0.5): in the above example, the cardiac signal exhibits two labels;
- computing the output of the network for this cardiac signal;
- modifying the parameters of the network so as to decrease a cost function comparing the known label vector and the output of the network; a cross-entropy error function is used so as to allow for multi-labeling (allowing for multiple labels for a cardiac signal); this minimization can be done though a gradient step;
- repeating steps 1 to 4 at least once for each cardiac signal of the dataset;
- recovering the neural network.

This invention also provides a method for manufacturing a neural network for both the delineation and the categorization of a cardiac signal, by training it.

In the embodiment of the combination of delineation with multi-label classification, the manufacturing process includes the following steps:

- taking one cardiac signal from a dataset containing cardiac signals and their known labels; the cardiac signal must be expressed as a matrix of size **m** x **n** with m fixed and at a predefined frequency;
- expressing the labels as a vector of size q, with q the number of labels to identify; this vector could be [0; 1; 0; 0; 1; 0; 0; 0] for q=8; a 1 is set in the vector at the index corresponding to the labels which are present (i.e. above a predefined threshold): in the above example, the cardiac signal exhibits two labels;
- expressing the delineation of this cardiac signal under the form of a matrix Y of size **p** x **n** where **p** is the number of waves to identify; typically **p**=3, so as to identify P waves, QRS waves, and T waves; annotations are usually expressed as lists wave type with their start and end points such as for example: (P, 1.2s, 1.3s), (QRS 1.4s 1.7s), (T, 1.7, 2.1), (P, 2.2, 2.3) ; in this example, the first row of Y, corresponding to P waves, will be 1 for samples corresponding to times between 1.2 and 1.3s, and between 2.2 and 2.4s, and 0 otherwise; row 2 will correspond to QRS complexes and row 3 to T waves;
- computing both outputs of the network for this cardiac signal;

- modifying the parameters of the network so as to decrease the sum of a cost function comparing the known label vector and one of the output of the network, and a cost function comparing the delineation and the other output; cross-entropy error functions are used to allow for multi-labeling (allowing for multiple labels for a cardiac signal as well as multiple waves at any time point); this minimization can be done though a gradient step;
- repeating steps 1 to 4 at least once for each cardiac signal of the dataset;
- recovering the neural network.

According to one embodiment, the step of expressing the delineation of the cardiac signal under the form of a matrix Y of size **p** x **n** further comprises wave characterization. According to said embodiment, **p** is the number of annotated types of wave plus the number of wave characterizations; for instance **p**=3+6=9 for identifying P waves, QRS complexes, and T waves, and characterizing premature waves, paced waves, ventricular QRS complexes, junctional QRS complexes, ectopic P waves and non-conducted P waves. According to said embodiment, annotations are expressed as lists of wave with their start and end points and characteristics such as for example: (P, 1.2s, 1.3s, [non-conducted]), (QRS 1.4s 1.7s, [premature, ventricular]), (T, 1.7, 2.1), (P, 2.2, 2.3); in this example, the first row of y, corresponding to P waves, will be 1 for samples corresponding to times between 1.2 and 1.3s, and between 2.2 and 2.4s, and 0 otherwise; row 2 will correspond to QRS complexes, row 3 to T waves, and row 4 corresponding to the premature characterization will be 1 during the premature QRS complex and 0 otherwise.

This invention also pertains to a method and a device for delineation of a cardiac signal, implementing a convolutional neural network, preferably a fully convolutional neural network, trained for delineation of a cardiac signal as described above.

As a basis, it shall be understood that the cardiac signal is expressed as a matrix X of size **m** x **n** at the frequency used for training the networks. The cardiac signal is used as input of the trained neural network.

The neural network then reads each time point of the cardiac signal, analyzes spatio-temporally each time point of the cardiac signal, assigns a temporal interval score to anyone of at least the following: P-wave, QRS complex, T-wave. It then recovers the output of the neural network, as a matrix Y of size **p** x **n**. An example is shown in Figure 4: the first signal shows one of the leads of the ECG (to help the visualization), the following 3 signals are the outputs of the network, providing scores for P waves, QRS waves and T waves. As it can be seen, these scores are synchronized with the appearance of the aforementioned waves in the signal. According to one embodiment, when multiple leads cardiac signal is used, all the leads are processed simultaneously.

In a preferred embodiment, the neural network provides scores at each time point as a matrix Y, and a post-processing allows the allocation of each time point to none, single, or several waves, and provides the onset and offset of each of the identified waves as well as optionally its characterization. For instance, a sample can be affected to the waves for which the score on the corresponding row of Y is larger than 0.5. Wave characterization such as conductivity, prematurity and origin of the wave can be recovered from the activation of the corresponding row between the onset and the offset of the wave. The premature label can for instance be applied to the wave if the average of the row corresponding to the premature characterization is above 0.5 during the wave. This provides a delineation sequence of type (P, 1.2s, 1.3s, [non-conducted]), (QRS 1.4s 1.7s, [premature, ventricular]), (T, 1.7s, 2.1s), (P, 2.2s, 2.3s), as recorded in the annotations.

The invention also comprises a computer device implemented software comprising a trained neural network for delineation of a cardiac signal. The invention also comprises a device, such as for example a cloud server, a commercial ECG device, a mobile phone or a tablet, comprising a software implementing the method for delineation as described above.

According to one embodiment, the device further comprises a display configured for displaying the wave locations and optionally simultaneously the cardiac signal.

According to one embodiment, global measurements derived from the delineation sequence such as for instance the PR interval are displayed. According to one embodiment, global measurements derived from the delineation sequence are highlighted for values which are not in a normal range. According to one embodiment, local measurements such as for instance all RR intervals are displayed with the cardiac signal. According to one embodiment, the conduction pattern of the cardiac signal is displayed in order to easily visualize characterization such as for instance prematurity of the waves with the cardiac signal. In an embodiment, the waves are displayed according to time with the cardiac signal.

This invention also pertains to a method and a device for multi-label classification of a cardiac signal, implementing Long-term Recurrent Convolutional Networks (LRCN, (Donahue et al., arXiv:1411.4389v3, 17 Feb 2015). These neural networks are trained for multi-label classification of a cardiac signal as described above.

As a basis, it shall be understood that the cardiac signal is expressed as a matrix of size m x n at the frequency used for training the networks. Then, the cardiac signal is used as input of the trained neural network.

The neural network then reads each time point of the cardiac signal, analyzes temporally each time point of the cardiac signal, computes a score for each label, recovers the output of the neural network. In an embodiment, the labels are non-exclusive.

In an embodiment, some other information can be included as inputs of the network. Said information can be delineation-derived such as for instance PR interval duration, heart rate, ST elevation or amplitudes of the QRS waves. It can also be patient-based such as their age or any relevant clinical information.

In an embodiment, the neural network NN2 reads and analyzes each time point of the cardiac signal and further the global measurements obtained from NN1.

In a preferred embodiment, the neural network recovers the output as a vector of size q. This vector contains scores for the presence of each label. According to one embodiment, a label is considered as present if its score is above a predefined threshold. This threshold is usually set to 0.5. It can however be modified to provide a different sensitivity-specificity couple. Indeed, increasing the threshold leads to lower specificity and higher specificity, and conversely when decreasing it. This set of couples is called a receiver operating characteristics curve and any point of this curve can be chosen through a modification of the threshold.

The invention also comprises a computer device implemented software comprising a trained neural network for multi-label classification of a cardiac signal. The invention also comprises a device, such as for example a cloud server, a commercial ECG device, a mobile phone or a tablet, comprising a software implementing the method of multi-label classification of a cardiac signal as described above.

According to one embodiment, the device further comprises a display configured for displaying the scores of the labels which have been allotted to a time window and optionally simultaneously the cardiac signal.

According to an embodiment, the list of found labels for which the score in the vector are higher than a predefined threshold, typically 0.5 is displayed. Labels can also be added depending on the delineation (delineation-based label), such as for instance the label corresponding to first degree atrioventricular block which is equivalent to a PR interval longer than 200ms, said PR interval being a global measurement based on the delineation. The list of labels can finally be filtered to remove redundant labels based on a known hierarchy of labels (for instance only the most detailed labels are retained), or aggregated through time on long cardiac signal so as to recover the start and end times of each abnormality.

This invention also pertains to a method and a device for delineation and multi-label classification of a cardiac signal, implementing a neural network trained for delineation and multi-label classification of a cardiac signal as described above.

As a basis, it shall be understood that the cardiac signal is expressed as a matrix of size **m** x **n** at the frequency used for training the networks. Then, the cardiac signal is used as input of the trained neural network.

The neural network then reads each time point of the cardiac signal, analyzes temporally each time point, assigns a temporal score to all of the following at least: P-wave, QRS complex, T-wave. It then computes a score for each labels, recovers both the outputs of the neural network: the first as a matrix y of size **p** x **n**, providing scores for at least P waves, QRS waves and T waves; and the second as a vector of size q, said vector containing scores for the presence of each label.

In a preferred embodiment, a post-processing of the delineation output allows to affect each time point to none, single, or several waves, and provides the onset and offset of each of the identified waves. For instance, a sample can be affected to the waves for which the score on the corresponding row of Y is larger than 0.5. This provides a delineation sequence of type (P, 1.2s, 1.3s), (QRS 1.4s 1.7s), (T, 1.7s, 2.1s), (P, 2.2s, 2.3s), as recorded in the annotations.

According to an embodiment, the list of found labels for which the score in the vector are higher than a predefined threshold, typically 0.5, are displayed; as well as the delineation, optionally with the cardiac signal.

According to an embodiment of the invention, a step to prepare the signal and create input variables for classification is further carried out ("pre-treatment"). The purpose of this pre-treatment is to remove the disturbing elements of the signal such as for example noise and baseline, low frequency signal due to respiration and patient motion, in order to facilitate classification. For noise filtering, a multivariate approach functional analysis proposed by (Pigoli and Sangalli, Computational Statistics and Data Analysis, vol.56,2012, pp 1482-1498) can be used. The low frequencies of the signal corresponding to the patient's movements may be removed using median filtering as proposed by (Kaur et al., Proceedings published by International Journal of Computer Applications, 2011, pp 30 -36).

According to an embodiment of the invention, a post-treatment step is added, so as to produce the onset and offset of each wave in the cardiac signal.

The invention also comprises a computer device implemented software comprising a trained neural network for delineation and multi-label classification of a cardiac signal. The invention also comprises a device, such as for example a cloud server, a commercial ECG device, a mobile phone or a tablet, comprising a software implementing the method of delineation and multi-label classification of a cardiac signal as described above.

According to one embodiment, the device further comprises a display configured for displaying the wave locations, the scores of the labels which have been allotted to a time window and optionally simultaneously the cardiac signal.

In an embodiment, global and local measurements derived from the delineation sequence such as for instance the PR interval are displayed. In an embodiment, the global and local measurements derived from the delineation sequence are highlighted for values which are not in a normal range. In an embodiment, the conduction pattern of the cardiac signal is displayed in order to easily visualize characterization such as for instance prematurity of the waves; and the waves may be displayed according to time.

The present invention further relates to a system comprising an electrocardiograph for recording cardiac signal and for implementing the methods according to the present invention. Thus, the electrocardiograph provides labels, delineation, measurements and conduction pattern of the cardiac signal right after the recording.

This invention brings to the art a number of advantages, some of them being described below:

- The input of the networks are one or multi-lead cardiac signals with variable length, possibly preprocessed so as to remove noise and baseline wandering due to patients movements, and express the signal at a chosen frequency.

- Using the presented lead invariant structure, a same network can handle cardiac signals with different number of leads.

- The output of a classification network is a vector of scores for labels. These are not classification scores since one cardiac signal can present several labels. For example, the output of such network could be a vector [0.98; 0.89; 0.00; ...] with the corresponding labels for each element of the vector (Right Bundle Branch Bloc; Atrial Fibrillation; Normal ECG; ...). Scores are given between a scale of [0, 1] and the above example output vectors therefore indicates a right bundle branch block and atrial fibrillations. A recurrent neural network architecture can be added on the top of the convolutional network (Donahue et al., arXiv:1411.4389v3, 17 Feb 2015 and Mnih et al., arXiv:1406.6247v1, 24 Jun 2014). In this way, the convolution network acts as a pattern detector whose output will be accumulated in time by the recurrent network.

- The output of the delineation network is a set of signals spanning the length of the input cardiac signal, providing the score for being in waves such as for instance P waves, QRS complexes, a T waves and potentially other types of waves or segments such as for example flutter waves, U waves or noisy segments. An example of output signals is provided in Figure 5.

- The delineation network can also characterize the waves such as for instance their prematurity, conductivity and ectopy. This ability allows to unify two steps of the delineation which are separate in current methods, making the proposed method more reliable and able to leverage context information for the waves characterization.

- The delineation network is not limited to recovering at most one wave at each time point and therefore can identify several waves at any time point, such as for instance P waves hidden in a T wave.

- The delineation network allows both the recovery of the start and end of each wave and there characterization in a single step, which is more reliable than all previous methods. In particular in is more reliable to recover P waves and characterize them, allowing to provide the conductivity pattern of the cardiac signal.

- No works applying convolutional networks to the delineation have been made so far.

The underlying structure of the networks is not fundamental as long as they are convolutional neural networks. One can use a structure such as RLCN (Donahue et al., arXiv:1411.4389v3, 17 Feb 2015 and Mnih et al., arXiv:1406.6247v1, 24 Jun 2014) for classification and a network similar as the one in (Long et al., Proceedings of Computer Vision and Pattern Recognition, 2015, pp 3431-3440) for delineation. In both embodiments, convolutional layers must be modified as 1D convolutions instead of 2D convolutions. On top of these architectures, both embodiments can use a lead invariant structure such as but not limited to the one presented in figure 8.

A hybrid network, sharing the first layers and diverging so as to provide both the delineation as one output, and the multi-label classification as another output is also used. This combination has the advantage of being able to produce a multi-label classification helped by the identification of the cardiac signal waves.

## EXAMPLES

The neural networks used within the present invention, were filed at LOGITAS under number D16201.

The present invention is further illustrated by the following examples.

Example 1: Training for delineation

This training was performed on 2204 ECGs and the network evaluated on about 900 beats from 77 different patients which were not used for the training phase. The following table provides the precision of the wave onsets (beginnings) and offsets (ends) in term of bias and standard deviation (std) as well as the false positive (FP) and false negative (FN) rates of the waves detection and of their characterizations:

| | FP (%) | FN (%) | Bias (ms) | Std (ms) | Count |
|---|---|---|---|---|---|
| P | 7.9 | 5.6 | 0.9 | 10.6 | 730 |
| PQ | 0 | 0.3 | 0.3 | 7.2 | 616 |
| QRS | 0 | 0 | 1.8 | 5.2 | 887 |
| QT | 0 | 0.1 | 0.7 | 13.3 | 873 |
| P onset | N/A | N/A | -2.9 | 6.4 | 689 |
| P offset | N/A | N/A | -2 | 8.4 | 689 |
| QRS onset | N/A | N/A | -3.1 | 4 | 887 |
| QRS offset QT offset | N/A | N/A | -1.3 -2.3 | 3.6 12.3 | 887 872 |
| ectopic P | 6 | 16 | N/A | N/A | 75 |
| premature P | 0 | 20 | N/A | N/A | 10 |
| paced P | 0 | 24.3 | N/A | N/A | 37 |
| non-conducted P | 0 | 8.3 | N/A | N/A | 36 |
| ventricular QRS | 2.7 | 5.3 | N/A | N/A | 38 |
| premature QRS | 8.3 | 15.4 | N/A | N/A | 26 |
| paced QRS | 0 | 0 | N/A | N/A | 22 |
| junctional QRS | 0 | 14.6 | N/A | N/A | 48 |

Concerning hidden P waves, the proposed algorithm was able to recover 75 out of 87 hidden P waves present in this evaluation dataset, while other algorithms would not be able to find any of them.

From the onsets and offsets of each wave are derived standard global measurements such as the P duration, PR interval, QRS duration and QT interval. An evaluation was performed on the standard CSE dataset which provides acceptance limits for delineation algorithms (Christov et al. BioMedical Engineering OnLine, 2006, vol. 5, pp. 31-38), yielding the following results which are well within the acceptance range:

| Measurement | Standard Deviation (ms) | | Bias (ms) | |
|---|---|---|---|---|
| | Result | Limit | Result | Limit |
| **P** | 3.8 | 15 | -2.4 | 10 |
| **PQ** | 6.1 | 10 | 0.1 | 10 |
| **QRS** | 4.2 | 10 | 2.0 | 10 |
| **QT** | 7.2 | 30 | -14.2 | 25 |

The following table sums up the results on the MIT-BIH Arrhythmia Database (Moody et al, Computers in Cardiology, 1990, vol. 17, pp.185-188) of a delineation network with a lead-invariant structure, which was not used for the training, in terms of QRS and premature ventricular complexes (PVC) detections:

| | FP (%) | FN (%) | Count |
|---|---|---|---|
| QRS | 0.32 | 0.17 | 107341 |
| PVC | 7.68 | 15.10 | 7071 |

Compared with state-of-the-art algorithms, the precision was improved and the ability of the algorithm, which can find the waves and characterize them at the same time, is much more efficient. In Figure 5 for instance, the ECG exhibits an atrioventricular block which means that the P waves and the QRS complexes are completely decoupled. In this example, the P waves are regular and QRS complexes happen at random times. One can observe in this example that the algorithm correctly found two P waves between the first QRS complex and the second QRS complex, while most algorithms would not be able to find them since they look for only one P wave before each complex. The last P wave also starts before the end of the last T wave, adding complexity. Finally, the algorithm is able to characterize theses waves as non-conducted. Other algorithms would not have been able to find the hidden waves and would not have been able to characterize any wave as non-conducted.

Example 2: Training for multi-label classification

A network has been trained using about 85,000 ECGs and has been evaluated on a dataset representative of a hospital emergency unit including 1,000 patients which were not used in the training phase. The results in terms of accuracy, specificity, sensitivity, and positive predict values were the following for some of the searched labels:

| | Population | Accuracy | Sensitivity | Specificity | PPV |
|---|---|---|---|---|---|
| Normal ECG | 421 | 77.39% | 66.75% | 89.06% | 87.00% |
| Atrial fibrillation | 22 | 99.75% | 90.91% | 100.00% | 100.00% |
| Atrial flutter | 3 | 99.88% | 66.67% | 100.00% | 100.00% |
| Junctional rhythm | 5 | 99.75% | 80.00% | 99.88% | 80.00% |
| Pacemaker | 5 | 100.00% | 100.00% | 100.00% | 100.00% |
| Premature ventricular complex(es) | 17 | 99.63% | 88.24% | 99.87% | 93.75% |
| Complete right bundle branch block | 21 | 99.50% | 90.48% | 99.74% | 90.48% |
| Complete left bundle branch block | 4 | 99.75% | 75.00% | 99.88% | 75.00% |
| Left ventricular hypertrophy | 7 | 99.38% | 57.14% | 99.75% | 66.67% |
| Acute STEMI | 5 | 100.00% | 100.00% | 100.00% | 100.00% |
| Old MI | 27 | 93.79% | 70.37% | 94.60% | 31.15% |

A neural network with a lead-invariant structure aimed at classifying rhythm abnormalities was also trained. Its performance on Holter ECGs in term of atrial fibrillation was analyzed on the MIT-BIH Arrhythmia Database (Moody et al, Computers in Cardiology, 1990, vol. 17, pp.185-188) comprising 30 minutes 2-lead ECGs of 48 different patients. To this end, the neural networks analyzed all 20 second segments of the ECG, which providing a rhythm label each 20 second, which were aggregated to provide the beginning and end of each rhythm abnormality or descriptor. The recovered labels were compared to the reference annotations, yielding a, accuracy, sensitivity, specificity and positive predictive value (PPV) for the atrial fibrillation label and the less specific atrial fibrillation or flutter label:

| | Accuracy | Sensitivity | Specificity | PPV |
|---|---|---|---|---|
| Atrial fibrillation | 98.3% | 96.9% | 98.5% | 89.6% |
| Atrial fibrillation or flutter | 99.0% | 96.8% | 99.2% | 92.3% |

These results are similar to the state-of-the-art in term of sensitivity, but significantly better than state-of-the-art methods in term of specificity and therefore also in accuracy and PPV.

A graphical representation of how a standard multi-label is used on ECGs is displayed in Figure 6. The ECG is given as input to the network, which aggregates the information locally and then combines it layer by layer to produce a high-level multi-label classification of the ECG, in this example correctly recognizing atrial fibrillations and a right bundle branch block. Such networks however take a fixed size as input and the process must be reproduced at different locations so as to analyze the whole signal. Figure 7 is an example of a graphic representation of a recurrent neural network which overcomes this issue. This type of network is made from a standard convolutional network computed at all possible locations of the signal, and on top of which comes another network layer which accumulates the information. In this example, the network correctly recognizes a premature ventricular complex (PVC, the fifth and largest beat) in the first part of the signal while the second part of the signal is considered as normal. The accumulated output is therefore PVC since this ECG has an abnormality and cannot therefore be considered as normal.

Example 3: Delineation and multi-label classification

In another embodiment, the applicant combines features described above in examples 1 and 2. Such combination enables to combine the advantages of both networks in a unique network, providing similar results for both the delineations and the multi-label classifications.

Example 4: Platform use case

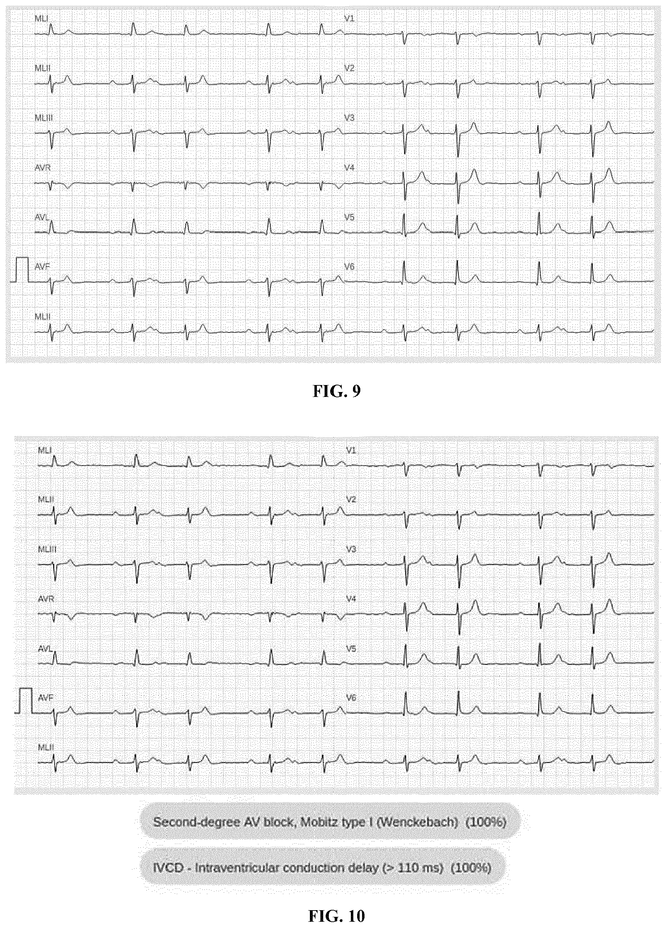

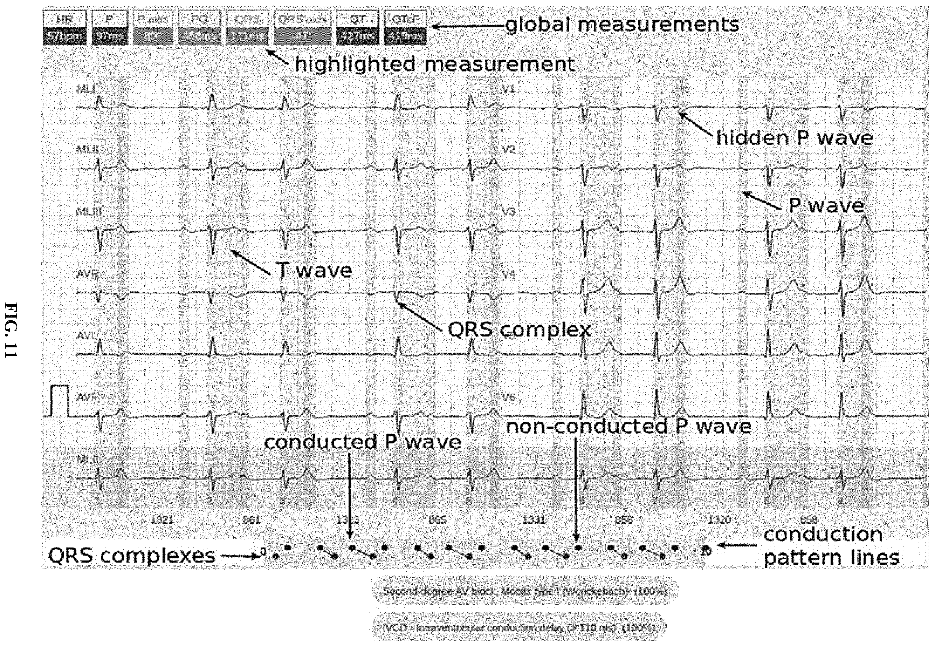

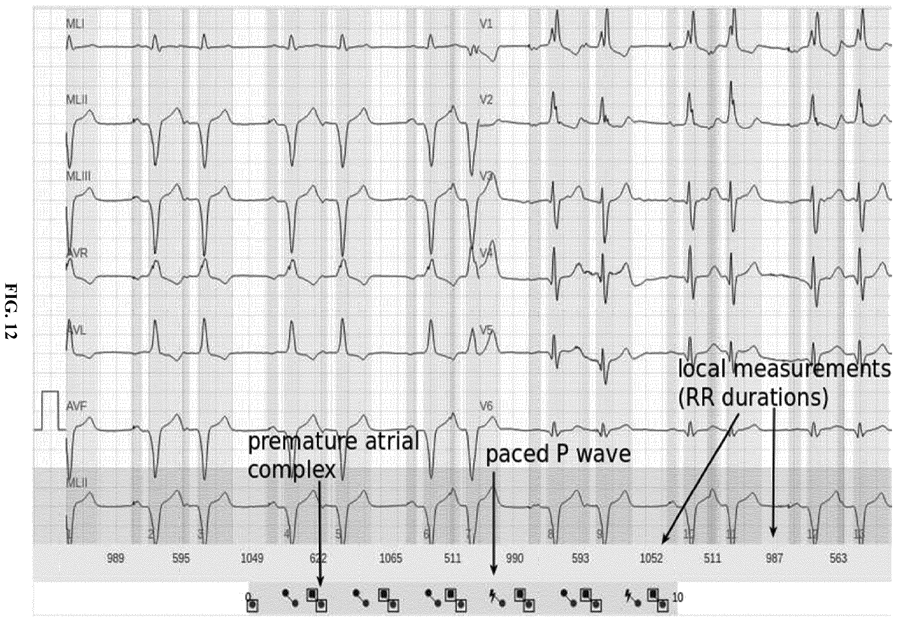

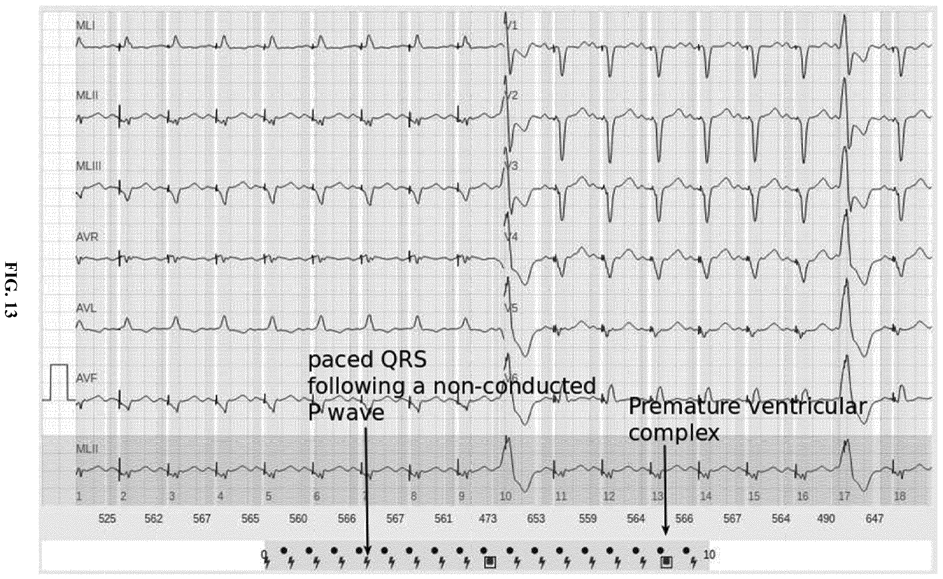

According to one embodiment, a user can log into a web platform. An upload button is available for the user to upload one of their ECGs in a supported formal so as to process it. The user is then redirected to a page displaying the ECG as shown in Figure 9. The user can than select to show the detected abnormalities as shown in Figure 10. In this case, the neural networks correctly detected a second degree atrioventricular block Mobitz I (a lengthening of the PR interval leading to a non-conducted P wave) and an intraventricular block (causing a lengthened QRS duration). The user can also choose to display the delineation information as shown in Figure 11. This information includes highlighting of the identified waves on the ECG drawing, printing global measurements derived from the delineation above the ECG drawing such as for example heart rate (HR) and QRS duration (QRS), with highlighting of the values which are not in a normal range such as for instance a QRS duration larger than 110ms. Local measurements such as all RR intervals are also shown as figures under the ECG. It also includes a laddergram-like feature showing the conduction pattern of the waves, their prematurity and their origin. This feature is displayed under the ECG, with each dot on the first line being a P wave, each dot of the second wave being a QRS wave and lines between them implying conduction. One can observe in this case that some P waves are not conducted. These same P waves are hidden P waves since they occur during T waves. In figures 12 and 13, one can see different examples of the conduction pattern display where the prematurity of the P and QRS waves are shown (with surrounding squares), and the origin of the waves are also shown (lightning bolt for a paced wave, square for ectopic P wave or ventricular QRS complex, triangle for junctional QRS complex etc).

Example 5: Application Programming Interface (API) use case

According to an embodiment, a user can also send an ECG through an API. The ECG is received on the platform and analyzed. The user can then recover information such as the delineation and the multi-label classification through another API.

Example 6: Resting ECG interpretation

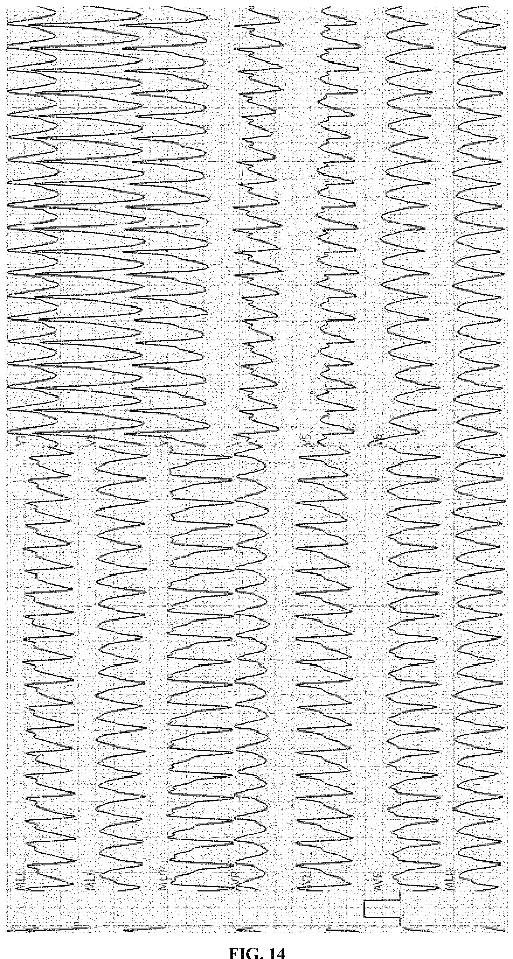

A patient arrives at the emergency unit of a hospital and an ECG is performed. The ECG shows wide complex tachycardia. Such a pattern can occur in very different situations, such as in the case of ventricular tachycardia, or with both atrial fibrillation and Wolff-Parkinson-White syndrome, or with both a bundle branch block and sinus tachycardia. Such conditions must be treated differently, the two former being life-threatening. Standard algorithms of the prior art can only detect one abnormality at a time and not a combination of labels. In this case, it is however crucial to be able to perform multi-label classification since interpretations may imply a combinations of labels. Being able to do so help properly identifying an actual ventricular tachycardia that other algorithms have difficulty to identify such as the one in figure 14 Indeed, method according to the present invention is able to test all other possible combinations of labels and rule them out.
Also, during an examination a general practitioner performs an ECG on a patient. The delineation is then helpful in order to highlight hidden P waves which may completely change the diagnostic between normal sinus rhythm and a 2nd degree atrioventricular block which may require the use of a pacemaker.

Example 7: Holter interpretation

A patient is prescribed a 7 day Holter. The 7 days must afterwards be interpreted by a specialist. The proposed algorithm is able to identify noisy segments of the signal which are common in Holters since the patient is allowed to move. It can also find atrial fibrillation or atrial flutter which is often looked at in Holters. Thanks to its multi-label ability, the proposed algorithm can also find atrial fibrillation during noise segments. In other situations, the patient could be monitored at a hospital in order to assess the possibility of an acute myocardial infarction. The proposed method can then provide ST elevations through time thanks to the delineation (amplitude at the QRS offset minus amplitude at the QRS onset) which changes are a very important indicator of STEMI (ST elevation myocardial infarction).

**BRIEF DESCRIPTION OF THE DRAWINGS**

**Figure** 1 is a photo of an ECG.

**Figure** 2 is a schematic representation of a normal ECG, with the P wave, the QRS complex/wave comprising the Q, R, S and J points, and the T wave.

**Figure** 3 is an example of structure for a basic neural network with no convolutional layer.

**Figure** 4 is an example of the output of the delineation network on a normal ECG.

**Figure** 5 is an example of the output of the delineation network on an ECG with hidden P waves (high degree atrioventricular block).

**Figure** 6 models the way a standard multi-label convolutional network works.

**Figure** 7 models the way a multi-label recurrent convolutional network works.

**Figure** 8 provides an example of structure to use as first layers of a neural network to make it able to process any number of leads.

**Figure** 9 shows the interface after loading an ECG.

**Figure 10** shows the interface with the ECG and the labels provided by the multi-label classification algorithm.

**Figure** 11 shows the interface with the ECG, the labels provided by the multi-label classification algorithm, the delineation of the waves on the ECG, measurements derived from the delineation above the ECG with highlighted abnormal values, and conduction pattern under the ECG derived from the delineation. Some waves, including one hidden P wave are more specifically shown.

**Figure 12** shows an example ECG with its conduction pattern comprising premature atrial complexes and one paced P waves. This conduction pattern is composed of a first line with the P waves as dots, and a second line with the QRS as dots. It can be synchronized or not with the signal depending on its use (not synchronized on this example).

**Figure 13** shows an example ECG with its conduction pattern comprising non-conducted P waves, paced QRS complexes and premature ventricular complexes. These elements are pointed at on the figure.

**Figure 14** shows an ECG with a ventricular tachycardia which was not identified by a prior art algorithm but is correctly identified by the proposed algorithm.

**Claims**

**1.** A computer implemented method for delineation and multi-label classification of a cardiac signal having a plurality of time points, said method comprising:

- receiving the cardiac signal, the cardiac signal generated by a plurality of electrocardiogram (ECG) leads sensing over a plurality of heartbeats;
- applying a first convolutional neural network to said cardiac signal, wherein the first convolutional neural network:

• reads each time point of the plurality of time points assigned to the cardiac signal;
• analyzes spatio-temporally each time point of the cardiac signal;
• assigns to each time point of the cardiac signal a plurality of first scores indicative of the presence of a plurality of types of waves comprising at least the following types of waves: P-waves, QRS complexes, T-waves;
• computes an onset and an offset of each type of wave of the plurality of types of waves in the cardiac signal based on the plurality of first scores assigned to each time point;
• computes a plurality of global measurements based on the onsets and the offsets; and

- applying a second convolutional neural network to said cardiac signal, wherein the second convolutional neural network:

• reads each time point of the cardiac signal and the plurality of global measurements;
• analyzes each time point of the cardiac signal and analyzes the plurality of global measurements;
• computes a plurality of second scores for a time window aggregating at least two time points; and
• allots to the time window a plurality of labels which each have a score higher than at least one predetermined threshold.

2. The method according to claim 1, wherein the cardiac signal is expressed as a matrix of size m x n and the output of the first convolutional neural network is expressed as p x n, where m is a number of leads, n is a number of samples, and p is a number of the types of waves in the plurality of types of waves plus a number of wave characterizations.

3. The method according to any of claim 1 or 2, wherein the types of waves further comprise hidden P-waves.

4. The method according to any of the preceding claims, wherein the wave characterizations comprise at least one of the following: premature waves, paced waves, ventricular QRS complexes, junctional QRS complexes, ectopic P waves, and non-conducted P waves.

5. The method according to any of the preceding claims, further comprising prior to applying the first convolutional neural network to the cardiac signal pre-treating the cardiac signal by denoising and removing a baseline of the cardiac signal as well as expressing the cardiac signal at a frequency.

6. The method according to any of the preceding claims, wherein the global measurements comprise at least one of the following: a mean or median value of a PR interval, a P duration, a QRS duration, a QRS axis, a median QT interval, a corrected QT interval, a corrected JT interval, a heart rate, a ST elevation, a Sokolov index, a premature ventricular complex, a premature atrial complex, a ratio of non-conducted P-waves, or a ratio of paced waves.

7. The method of any of the preceding claims, further comprising pre-treating the cardiac signal prior to applying the second convolutional neural network to the cardiac signal by denoising the cardiac signal, removing a baseline of the cardiac signal, and expressing the cardiac signal at a frequency.

8. The method according to any of the preceding claims, wherein the plurality of labels includes at least two of the following labels: sinoatrial block, paralysis, or arrest; atrial fibrillation; atrial flutter; atrial tachycardia; junctional tachycardia;
supraventricular tachycardia; sinus tachycardia; ventricular tachycardia; pacemaker; premature ventricular complex; premature atrial complex; first degree atrio-ventricular block (AVB); second degree AVB Mobitz I; second degree AVB Mobitz II; third degree AVB; Wolff-Parkinson-White syndrome; left bundle branch block; right bundle branch block; intraventricular conduction delay; left ventricular hypertrophy; right ventricular hypertrophy; acute myocardial infarction; old myocardial infarction; ischemia; hyperkalemia; hypokalemia; Brugada syndrome; or long QTc.

9. The method of any of the preceding claims, further comprising post-treating the cardiac signal by removing redundant labels.

10. The method of any of the preceding claims, wherein the second convolutional neural network is a recurrent convolutional neural network.

11. The method of any of the preceding claims, wherein the first convolutional neural network is a fully convolutional neural network.

**Patentansprüche**

1. Computerimplementiertes Verfahren zur Darstellung und Multi-Label-Klassifikation eines Herzsignals mit mehreren Zeitpunkten, wobei das Verfahren aufweist:

   - Empfangen des Herzsignals, wobei das Herzsignal durch mehrere Elektrokardiogramm- (EKG-) Leitungen, die über mehrere Herzschläge abtasten, erzeugt wird;
   - Anwenden eines ersten Faltungsneuronalnetzwerks auf das Herzsignal, wobei das erste Faltungsneuronalnetzwerk:

     • jeden Zeitpunkt der mehreren Zeitpunkte, die dem Herzsignal zugewiesen sind, liest;
     • jeden Zeitpunkt des Herzsignals räumlich-zeitlich analysiert;
     • jedem Zeitpunkt des Herzsignals mehrere erste Punktzahlen zuordnet, welche das Vorhandensein mehrerer Arten von Wellen anzeigen, die wenigstens die folgenden Wellenarten aufweisen: P-Wellen, QRS-Komplexe, T-Wellen;
     • einen Anfang und einen Versatz jeder Wellenart der mehreren Wellenarten in dem Herzsignal basierend auf den mehreren ersten Punktzahlen, die jedem Zeitpunkt zugeordnet sind, berechnet;
     • basierend auf den Anfängen und Versätzen mehrere globale Messungen berechnet; und

   - Anwenden eines zweiten Faltungsneuronalnetzwerks auf das Herzsignal, wobei das zweite Faltungsneuronalnetzwerk:

     • jeden Zeitpunkt des Herzsignals und die mehreren globalen Messungen liest;
     • jeden Zeitpunkt des Herzsignals analysiert und die mehreren globalen Messungen analysiert;
     • mehrere zweite Punktzahlen für ein Zeitfenster, das mindestens zwei Zeitpunkte vereinigt, berechnet; und
     • dem Zeitfenster mehrere Label zuweist, die jeweils eine höhere Punktzahl als mindestens ein vorgegebener Schwellwert haben.

2. Verfahren nach Anspruch 1, wobei das Herzsignal als eine Matrix einer Größe m x n ausgedrückt wird, und die Ausgabe des ersten Faltungsneuronalnetzwerks als $p \times n$ ausgedrückt wird, wobei m eine Anzahl von Leitungen ist, n eine Anzahl von Proben ist, und p eine Anzahl der Wellenarten der mehreren Wellenarten plus eine Anzahl von Wellencharakterisierungen ist.

3. Verfahren nach einem der Ansprüche 1 oder 2, wobei die Wellenarten ferner verborgene P-Wellen aufweisen.

4. Verfahren nach einem der vorhergehenden Ansprüche, wobei die Wellencharakterisierungen mindestens eine der folgenden aufweisen: vorzeitige Wellen, Schrittmacherwellen, ventrikuläre QRS-Komplexe, junktionale QRS-Komplexe, ektopische P-Wellen und nicht leitungsgebundene P-Wellen.

5. Verfahren nach einem der vorhergehenden Ansprüche, das vor dem Anwenden des ersten Faltungsneuronalnetzwerks auf das Herzsignal die Vorbehandlung des Herzsignals durch Rauschunterdrücken und Entfernen der Basislinie des Herzsignals ebenso wie Ausdrücken des Herzsignals bei einer Frequenz aufweist.

6. Verfahren nach einem der vorhergehenden Ansprüche, wobei die globalen Messungen wenigstens eines der folgenden aufweisen: einen Mittel- oder Medianwert eines PR-Intervalls, eine P-Dauer, eine QRS-Dauer, eine QRS-Achse, ein Median-QT-Intervall, ein korrigiertes QT-Intervall, ein korrigiertes JT-Intervall, eine Herzfrequenz, eine ST-Hebung, einen Sokolov-Index, einen vorzeitigen ventrikulären Komplex, einen vorzeitigen atrialen Komplex, ein Anteil nicht leitungsgebundener P-Wellen, oder ein Anteil von Schrittmacherwellen.

7. Verfahren nach einem der vorhergehenden Ansprüche, das ferner die Vorbehandlung des Herzsignals vor dem Anwenden des zweiten Faltungsneuronalnetzwerks auf das Herzsignal durch Rauschunterdrücken und Entfernen der Basislinie des Herzsignals ebenso wie Ausdrücken des Herzsignals bei einer Frequenz aufweist.

8. Verfahren nach einem der vorhergehenden Ansprüche, wobei die mehreren Label wenigstens zwei der folgenden Label umfassen: sinuatrialer Block, Paralyse oder Arrest; atriale Fibrillation; atriales Flimmern; atriale Tachykardie; junktionale Tachykardie; supraventrikuläre Tachykardie; Sinustachykardie; ventrikuläre Tachykardie; Schrittmacher, vorzeitiger ventrikulärer Komplex; vorzeitiger atrialer Komplex; atrio-ventrikulärer Block ersten Grades (AVB); AVB-Mobitz-I zweiten Grades; AVB-Mobitz-II zweiten Grades; AVB dritten Grades; Wolff-Parkinson-White-Syndrom;

Linksschenkelblock; Rechtsschenkelblock; intraventrikuläre Leitungsverzögerung, Linksschenkel-Hypertrophie; Rechtsschenkel-Hypertrophie; akuter Myokardininfarkt; alter Myokardininfakrt; Ischämie; Hyperkaliämie; Hypokaliämie; Brugada-Syndrom; oder eine lange QTc.

**9.** Verfahren nach einem der vorhergehenden Ansprüche, das ferner die Nachbehandlung des Herzsignals durch Entfernen redundanter Labels umfasst.

**10.** Verfahren nach einem der vorhergehenden Ansprüche, wobei das zweite Faltungsneuronalnetzwerk ein wiederkehrendes Faltungsneuronalnetzwerk ist.

**11.** Verfahren nach einem der vorhergehenden Ansprüche, wobei das erste Faltungsneuronalnetzwerk ein vollständiges Faltungsneuronalnetzwerk ist.

**Revendications**

**1.** Procédé mis en œuvre sur ordinateur pour la délimitation et la classification multi-marqueurs d'un signal cardiaque ayant une pluralité de temps, ledit procédé comprenant :

- la réception du signal cardiaque, le signal cardiaque étant généré par une pluralité de dérivations d'électrocardiogramme (ECG) détectant sur une pluralité de battements cardiaques ;
- l'application d'un premier réseau neuronal convolutif audit signal cardiaque, dans lequel le premier réseau neuronal convolutif :

• lit chaque temps de la pluralité de temps assignés au signal cardiaque ;
• analyse de façon spatio-temporelle chaque temps du signal cardiaque ;
• assigne à chaque temps du signal cardiaque une pluralité de premiers scores indicatifs de la présence d'une pluralité de types d'ondes comprenant au moins les types d'ondes suivants : ondes P, complexes QRS, ondes T ;
• calcule un début et un décalage de chaque type d'onde de la pluralité de types d'ondes dans le signal cardiaque sur la base de la pluralité de premiers scores assignés à chaque temps ;
• calcule une pluralité de mesures globales sur la base des débuts et des décalages ; et

- l'application d'un deuxième réseau neuronal convolutif audit signal cardiaque, dans lequel le deuxième réseau neuronal convolutif :

• lit chaque temps du signal cardiaque et la pluralité de mesures globales ;
• analyse chaque temps du signal cardiaque et analyse la pluralité de mesures globales ;
• calcule une pluralité de deuxièmes scores pour une fenêtre de temps agrégeant au moins deux temps ; et
• attribue à la fenêtre de temps une pluralité de marqueurs qui ont chacun un score supérieur à au moins un seuil prédéterminé.

**2.** Procédé selon la revendication 1, dans lequel le signal cardiaque est exprimé sous la forme d'une matrice de taille m x n et la sortie du premier réseau neuronal convolutif est exprimée par p x n, où m est un nombre de dérivations, n est un nombre d'échantillons, et p est un nombre des types d'ondes dans la pluralité de types d'ondes plus un nombre de caractérisations d'onde.

**3.** Procédé selon l'une quelconque des revendications 1 ou 2, dans lequel les types d'ondes comprennent en outre des ondes P cachées.

**4.** Procédé selon l'une quelconque des revendications précédentes, dans lequel les caractérisations d'onde comprennent au moins l'un des suivants : ondes prématurées, ondes de stimulation, complexes QRS ventriculaires, complexes QRS jonctionnels, ondes P ectopiques et ondes P non conduites.

**5.** Procédé selon l'une quelconque des revendications précédentes, comprenant en outre, avant l'application du premier réseau neuronal convolutif au signal cardiaque, le prétraitement du signal cardiaque par débruitage et élimination d'une ligne de base du signal cardiaque ainsi que l'expression du signal cardiaque à une fréquence.

6. Procédé selon l'une quelconque des revendications précédentes, dans lequel les mesures globales comprennent au moins l'un des suivants : une valeur moyenne ou médiane d'un intervalle PR, une durée P, une durée QRS, un axe QRS, un intervalle QT médian, un intervalle QT corrigé, un intervalle JT corrigé, une fréquence cardiaque, une élévation du segment ST, un indice de Sokolov, un complexe ventriculaire prématuré, un complexe auriculaire prématuré, un rapport d'ondes P non conduites, ou un rapport d'ondes de stimulation.

7. Procédé selon l'une quelconque des revendications précédentes, comprenant en outre le prétraitement du signal cardiaque avant l'application du deuxième réseau neuronal convolutif au signal cardiaque par débruitage du signal cardiaque, élimination d'une ligne de base du signal cardiaque, et expression du signal cardiaque à une fréquence.

8. Procédé selon l'une quelconque des revendications précédentes, dans lequel la pluralité de marqueurs comprend au moins deux des marqueurs suivants : bloc, paralysie ou arrêt sino-auriculaire, ; fibrillation auriculaire ; flutter auriculaire ; tachycardie auriculaire ; tachycardie jonctionnelle; tachycardie supraventriculaire; tachycardie sinusale ; tachycardie ventriculaire ; stimulateur cardiaque ; complexe ventriculaire prématuré ; complexe auriculaire prématuré ; bloc auriculo-ventriculaire (BAV) de premier degré ; BAV de deuxième degré Mobitz I ; BAV de deuxième degré Mobitz II ; BAV de troisième degré ; syndrome de Wolff-Parkinson-White; bloc de branche gauche ; bloc de branche droite ; retard de conduction intraventriculaire; hypertrophie ventriculaire gauche ; hypertrophie ventriculaire droite ; infarctus du myocarde aigu ; infarctus du myocarde ancien ; ischémie ; hyperkaliémie ; hypokaliémie ; syndrome de Bragada ; ou QTc long.

9. Procédé selon l'une quelconque des revendications précédentes, comprenant en outre le post-traitement du signal cardiaque par élimination des marqueurs redondants.

10. Procédé selon l'une quelconque des revendications précédentes, dans lequel le deuxième réseau neuronal convolutif est un réseau neuronal convolutif récurrent.

11. Procédé selon l'une quelconque des revendications précédentes, dans lequel le premier réseau neuronal convolutif est un réseau neuronal totalement convolutif.

**FIG. 1**

**FIG. 2**

**FIG. 3**

**FIG. 4**

**FIG. 5**

AFIB: 99%
RBBB: 98%
PVC: 0.1%
...

**FIG. 6**

FIG. 7

**FIG. 8**

**FIG. 9**

Second-degree AV block, Mobitz type I (Wenckebach) (100%)

IVCD - Intraventricular conduction delay (> 110 ms) (100%)

**FIG. 10**

23

FIG. 11

**FIG. 12**

**FIG. 13**

**FIG. 14**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8903479 B
- US 20140148714 A
- US 8332017 B
- CN 104970789

**Non-patent literature cited in the description**

- **MARTINEZ et al.** *IEEE transactions on biomedical engineering,* April 2004, vol. 51 (4), 570-581
- **ALMEIDA et al.** *IEEE transactions on biomedical engineering,* August 2009, vol. 56 (8), 1996-2005
- **BOICHAT et al.** *Proceedings of Wearable and Implantable Body Sensor Networks,* 2009, 256-261
- **COAST et al.** *IEEE transactions on biomedical engineering,* September 1990, vol. 37 (9), 826-836
- **HUGHES et al.** *Proceedings of Neural Information Processing Systems,* 2004, 611-618
- **CHAZAL et al.** *IEEE Transactions on Biomedical Engineering,* 2004, vol. 51, 1196-1206
- **KIRANYAZ et al.** *IEEE Transactions on Biomedical Engineering,* 2016, vol. 63, 664-675
- Statement of Validation and Accuracy for the Glasgow 12-Lead ECG Analysis Program. *Physio Control,* 2009
- **SHEN et al.** *Biomedical Engineering and Informatics (BMEI),* 2010, vol. 3, 960-964
- **JIN ; DONG.** *Science China Press,* 2015, vol. 45 (3), 398-416
- **BISHOP.** Pattern Recognition and Machine Learning. Springer, 2006
- **ROSENBLATT.** *Psychological Review,* 1958, vol. 65 (6), 386-408
- **CYBENKO.** *Math. Control Signals Systems,* 1989, vol. 2, 303-314
- **FUKUSHIMA.** *Biol. Cybernetics,* 1980, vol. 36, 193-202
- **LECUN et al.** *Neural Computation,* 1989, vol. 1, 541-551
- **LONG et al.** *Proceedings of Computer Vision and Pattern Recognition,* 2015, 3431-3440
- **KRIZHEVSK et al.** *Proceedings of Neural Information Processing Systems,* 2012, 1097-1105
- **PIGOLI ; SANGALLI.** *Computational Statistics and Data Analysis,* 2012, vol. 56, 1482-1498
- **KAUR et al.** *Proceedings published by International Journal of Computer Applications,* 2011, 30-36
- **CHRISTOV et al.** *BioMedical Engineering OnLine,* 2006, vol. 5, 31-38
- **MOODY et al.** *Computers in Cardiology,* 1990, vol. 17, 185-188